# EUROPÄISCHE PATENTSCHRIFT

(11) **EP 2 325 293 B1**
(45) Veröffentlichungstag und Bekanntmachung des Hinweises auf die Patenterteilung: **06.06.2012**
(21) Anmeldenummer: 10000900.0
(22) Anmeldetag: 29.01.2010
(51) Int. Cl.: C12M 1/00

(54) **Anordnung und Verfahren zur dreidimensionalen Verteilung von elektromagnetischer Strahlung in einem flüssigen Medium**
Assembly and method for three-dimensional distribution of electromagnetic radiation in a fluid medium
Appareil et procédé de répartition tridimensionnelle de rayonnement électromagnétique dans un milieu liquide

(30) Priorität: 20.11.2009 EP 09014525; 01.12.2009 EP 09014875
(43) Veröffentlichungstag der Anmeldung: 25.05.2011
(73) Patentinhaber: Universität Duisburg-Essen, 45141 Essen (DE); SEE-O-TWO Patentgesellschaft mbH, 1130 Wien (AT)
(72) Erfinder: Franke, Hilmar, 49565 Bramsche (DE); Niederl-Schmidinger, Anneliese, 1230 Wien (AT); Stagl, Karl, 2353 Guntramsdorf (AT)
(74) Vertreter: Gesthuysen, von Rohr & Eggert

(56) Entgegenhaltungen:
- WO-A2-2008/135276
- DE-C- 460 612
- KR-A- 20040 025 203
- US-A1- 2005 239 182
- DATABASE WPI Week 200446 Thomson Scientific, London, GB; AN 2004-485505 XP002592692 & KR 2004 025 203 A (INHA FOUND) 24. März 2004 (2004-03-24)
- HASHIMOTO MICHINAO ET AL: "Flowing lattices of bubbles as tunable, self-assembled diffraction gratings." SMALL (WEINHEIM AN DER BERGSTRASSE, GERMANY) NOV 2006 LNKD- PUBMED:17192976, Bd. 2, Nr. 11, November 2006 (2006-11), Seiten 1292-1298, XP002592544 ISSN: 1613-6829
- RIZZUTI, L. AND YUE, P.L.: "The measurement of light transmission through an irradiated fluidised bed" CHEMICAL ENGINEERING SCIENCE, Bd. 38, Nr. 8, 1983, Seiten 1241-1249, XP002592545

## Beschreibung

Die vorliegende Erfindung betrifft eine Anordnung zur dreidimensionalen Verteilung von Licht in einem flüssigen Medium, mit einer Beleuchtungseinrichtung und mit einer Einrichtung zur Einleitung von Gas in das Medium. Darüber hinaus betrifft die vorliegende Erfindung ein Verfahren zur dreidimensionalen Verteilung von Licht in einem flüssigen Medium, wobei Gas in das Medium eingeleitet und das Medium beleuchtet wird. Dabei kann es sich insbesondere um ein Verfahren zur Erzeugung von Biomasse oder von Stoffwechselprodukten von vorzugsweise Photosynthese betreibenden Organismen, insbesondere zur Verwandlung von Kohlendioxid in Biomasse, handeln, wobei ein flüssiges, die Organismen aufweisendes Medium, insbesondere ein flüssiges Medium mit Algen, beleuchtet und ein Gas, insbesondere kohlendioxidhaltiges oder daraus bestehendes Gas, in das Medium eingeleitet wird, um das Wachstum und/oder die Vermehrung der Organismen anzuregen und Biomasse oder Stoffwechselprodukte zu bilden.

Die vorliegende Erfindung befaßt sich insbesondere mit der Nachahmung der Photosynthese zur Bildung von Kohlendioxid. Es ist bekannt, hierzu eine Algensuspension zu beleuchten, um das Wachstum der Algen anzuregen. Die Algen verbrauchen Kohlendioxid zum Wachstum und zur Bildung von Biomasse. Um möglichst alle Algen in der Algensuspension mit Licht und Kohlendioxid zu versorgen, kann die Algensuspension umgewälzt und/oder die Oberfläche der Algensuspension möglichst groß gestaltet werden. Dies kann jedoch anlagenmäßig und/oder verfahrenstechnisch bzw. energetisch aufwendig sein.

Aus der WO 2008/135276 A2 sind eine Anordnung und ein Verfahren zur dreidimensionalen Verteilung von Licht in einem flüssigen Medium, wie einer Algensuspension, und/oder zur Erzeugung von Biomasse bekannt, bei dem Licht, insbesondere Sonnenlicht, mittels lichtleitenden Fasern in einen Behälter mit dem Medium eingeleitet wird. Das Licht wird dreidimensional im Behalter verteilt. Hierzu enden die Fasern in verschiedenen Raumbereichen innerhalb des Behälters. Alternativ oder zusätzlich wird das Licht über Gasblasen eingeleitet. Die Einleitung bzw. Erzeugung der Gasblasen erfolgt vorzugsweise über hohle Fasern. Beim Einleiten des Gases in das Medium bilden sich Gasblasen, die eine Größe von 1 bis 10 µm, insbesondere 2 bis 5 µm, aufweisen können. Bei dieser Größe bilden die Gasblasen besonders geeignete Resonanzkörper für das aufzunehmende Licht. Die hohlen Fasern geben gleichzeitig Licht an ihren Enden in den Bereichen ab, in denen die Gasblasen gebildet werden. So können die Gasblasen quasi mit Licht gefüllt werden. Es wird eine Art Nebel oder Wolke leuchtender Gasblasen erzeugt. Problematisch ist, dass die Algen bevorzugt auf den das Licht in das Medium einleitenden Grenzflächen oder Oberflächen, wie den hohlen Fasern, die das Licht in das Medium einleiten, aufwachsen.

Der vorliegenden Erfindung liegt die Aufgabe zugrunde, eine Anordnung und ein Verfahren zur Verfügung zu stellen, wobei ein einfacher Aufbau, ein einfacher, kostengünstiger und/oder energiesparender Verfahrensablauf und eine hohe Effektivität ermöglicht wird bzw. werden, insbesondere wobei Grenzflächen zur Einleitung von Licht, auf denen Algen oder sonstige Organismen o. dgl. aufwachsen können, vermieden werden können.

Die vorgenannte Aufgabe wird durch eine Anordnung mit den Merkmalen von Patentanspruch 1 und durch ein Verfahren mit den Merkmalen von Patentanspruch 14 gelöst. Vorteilhafte Weiterbildungen sind Gegenstand der Unteransprüche.

Eine grundlegende Idee der vorliegenden Erfindung liegt darin, das in das Medium eingeleitete Gas nicht (ausschließlich) vereinzelt in Form von Gasblasen aufsteigen zu lassen, sondern eine Blasenkette zu erzeugen, wobei durch Beleuchtung der Blasenkette bzw. Einleitung von Licht in die Blasenkette eine insbesondere kontaktlose, tief in das Medium eindringende, gleichmäßige und/oder vollständige Beleuchtung des Mediums erreichbar ist. Wird in die Blasenkette Licht eingekoppelt, wirkt diese wie ein Lichtleiter, wobei eine hohe Lichtintensität in der Blasenkette über die Oberfläche der Blasen in das Medium gelangen und wobei die abgestrahlte Intensität infolge Mehrfachreflexion stark abgeschwächt werden kann, was sich vorteilhaft auf Stoffumsatz und Wachstum von Organismen auswirkt. Darüber hinaus wird über die Blasenkette gleichzeitig das Gas in das Medium eingebracht.

Um die Blasenkette in einfacher Weise zu erzeugen, wird eine rotatorische bzw. kreis- oder spiralförmige Bewegung des Mediums erzwungen bzw. erzeugt, wobei sich eine geradlinig entlang einer Rotationsachse des Mediums erstreckende Blasenkette ausbilden kann. Hierbei ist es so, dass die Gasblasen sich im Bereich der Rotationsachse sammeln und eine Blasenkette bilden. Es versteht sich, dass die Form und Größe der Blasenkette unter anderem abhängt von der Winkelgeschwindigkeit des bewegten Mediums. So kann es insbesondere beim Andrehen des Mediums auch möglich sein, dass sich eine Blasenkette bildet, die eine Spiralform aufweist. Blasenketten können grundsätzlich jedoch auch in nicht kreis- oder spiralförmig bewegten Medien gebildet werden.

Der Begriff "Blasenkette" soll zum Ausdruck bringen, dass die Blasen zumindest im Wesentlichen linear hintereinander in einer Reihe aufsteigen oder sich sogar miteinander verbinden. Jedoch soll der Begriff "Blasenkette" in einem weiteren Sinne vorzugsweise auch als Blasenstrudel oder Blasenwolke verstanden werden.

Vorzugsweise wird Sonnenlicht zur Beleuchtung des Mediums eingesetzt, auch wenn grundsätzlich künstlich erzeugtes Licht verwendet werden kann. Auch ist es möglich, dass das Medium mit nicht-sichtbarer elektromagnetischer Strahlung, beispielsweise mit Infrarot- oder ultravioletter Strahlung, bestrahlt wird. Die Begriffe "Beleuchtung" und "Licht" umfassen daher vorzugsweise in einem breiten Sinn auch die Bestrahlung mit oder Einleitung sonstiger elektromagnetischer Strahlung.

Vorzugsweise wird eine einreihige Blasenkette erzeugt, die durch eine Mehrzahl von im wesentlichen gleich großen oder unterschiedlich großen in einer Reihe hintereinanderliegenden Blasen gebildet wird. Grundsätzlich ist es aber auch möglich, dass die Blasenkette gebildet wird durch eine Mehrzahl von gleich- oder unterschiedlich großen Blasen, die in mehreren Reihen nebeneinanderliegend nach oben aufsteigen und einen schlauchförmigen mit Blasen gefüllten Lichtleitraum bilden, Über die aneinander angrenzenden Blasen der Blasenkette wird das Licht innerhalb des Mediums in einfacher Weise verteilbar.

Insbesondere im Zusammenhang mit einem Verfahren zur Erzeugung von Biomasse oder von Stoffwechselprodukten von vorzugsweise Photosynthese betreibenden Organismen, wie bei einem Verfahren zur Umwandlung von Kohlendioxid in Biomasse, wobei ein flüssiges Medium mit Algen verwendet und Licht in dem Medium dreidimensional verteilt wird und wobei Kohlendioxid enthaltendes oder daraus bestehendes Gas in Form von Gasblasen in das Medium eingeleitet wird, führt die erfindungsgemäß vorgesehene Beleuchtung des Mediums über wenigstens eine Blasenkette zu einer sehr effektiven Bildung von Biomasse und/oder Bindung von Kohlendioxid.

### dassdassdass

Die erzeugte Blasenkette kann sich zumindest im wesentlichen durchgängig bzw. unterbrechungslos von einer Gaszuführung vorzugsweise im Bodenbereich des Mediums bis hin zur Oberfläche des Mediums erstrecken. Dies ermöglicht in einfacher Weise eine Beleuchtung bzw. Beleuchtung der Blasenkette vorzugsweise von oben, gegebenenfalls aber auch von der Seite oder von unten. Das Licht wird dann über die Blasenkette als Lichtleiter in dem Medium verteilt. Dabei läßt es die Erfindung in Abhängigkeit von den Strömungsverhältnissen im Mediumbehälter auch zu, dass sich einzelne Blasen der Blasenkette kurzzeitig voneinander trennen, so dass die Blasenkette zumindest zeitweise unterbrochen ist. Der Begriff "Blasenkette" ist in einem bevorzugten Sinn auch generell dahingehend zu verstehen, dass es sich hier um einen optisch durchlässigeren, insbesondere länglichen Raumbereich bzw. Gasbereich in dem Medium handelt.

Vorzugsweise soll das Medium in Schichten strömen, die sich nicht vermischen, so dass bei vorzugsweise zumindest im wesentlichen konstanter Strömungs- oder Winkelgeschwindigkeit eine zumindest im wesentlichen laminare bzw. turbulenzarme Strömung vorliegt. Dadurch kann insbesondere eine geradlinige einreihige Blasenkette ausgebildet werden, die sich im wesentlichen durchgängig, d. h. unterbrechungslos, innerhalb des Mediums erstreckt.

Für die Ausbildung einer Blasenkette im Bereich der Rotationsachse eines kreis- oder spiralförmig bewegten Mediums kann es von Vorteil sein, eine Gaszufuhr im Bereich der Rotationsachse, vorzugsweise über einen Boden eines Mediumbehälters oder -tanks vorzusehen, Die Einleitung der Gasblasen kann dann im Bereich der tiefsten Stelle des Mediums erfolgen, so dass sich die Blasenkette über die gesamte Flüssigkeitssäule des Mediums erstrecken kann. Ergänzend oder alternativ kann das Gas jedoch auch seitlich über den Mediumbehälter bzw. -tank in das Medium eingeleitet werden.

Um eine Kontamination mit dem Medium, insbesondere eine-Benetzung mit dem Medium und damit ein Wachstum von in dem Medium enthaltenen Organismen auf der Beleuchtungseinrichtung in einfacher Weise zu verhindern, kann eine mit dem Medium kontaktlose Beleuchtung der Blasenkette von einem Bereich außerhalb des Mediums vorgesehen sein. Hierzu kann die Beleuchtungseinrichtung ausreichend weit von dem Medium beabstandet angeordnet sein, wobei beispielsweise eine Beleuchtung der Blasenkette von oben in den Austritt der Gasblasen aus dem Medium vorgesehen sein kann. Alternativ oder zusätzlich kann - insbesondere bei einem Mediumbehälter bzw. -tank mit transparenten Seitenwänden - auch eine Beleuchtung durch die Seitenwände und/oder von unten in die Blasenkette vorgesehen sein. Bei einer Beleuchtung von unten und von oben läßt sich insbesondere eine größere Behälter- bzw. Medienhöhe realisieren, da das Licht von entgegengesetzten Seiten in das Medium eindringen kann.

Die kontaktlose Beleuchtung der Gasblasen ist insbesondere dann von Vorteil, wenn das Medium Algen oder sonstige Organismen enthält, die sehr leicht auf Oberflächen anwachsen, die mit dem Medium in Kontakt kommen. Durch das Anwachsen von Algen auf transparenten durchstrahlten Oberflächen der Beleuchtungseinrichtung kann es bereits nach kurzer Zeit zu einer Abnahme der Transparenz und zu einer verringerten Beleuchtungsleistung kommen.

Aufgrund der Bewegung des Mediums kann sich die Blasenkette gegebenenfalls bewegen, so dass sich die Lage des Austrittsbereiches der Gasblasen an der Oberfläche des Mediums ändern kann. Erfindungsgemäß kann daher eine Erfassungseinrichtung zur Bestimmung oder Verfolgung des Austrittsbereiches der Blasenkette an der Oberfläche des Mediums vorgesehen sein kann. Die Erfassungseinrichtung kann Mittel zur optischen Bestimmung sowie Verfolgung des Austrittsbereiches aufweisen. Durch Bestimmung oder Verfolgung des Austrittsbereiches der Blasenkette läßt sich eine Beleuchtung der Blasenkette über den Austrittsbereich in einfacher Weise auch bei nichtruhender Blasenkette gewährleisten. Auch ist es möglich, dass die Erfassungseinrichtung die Größe und/oder die Form und/oder das Umfangsprofil des Austrittsbereiches und/oder das Auftreten mehrerer Austrittsbereiche erfaßt, so dass in einfacher Weise Rückschlüsse auf die Lage und den Verlauf der Blasenkette im Medium bzw. die Form und Größe der Blasenkette möglichsind.

Die Erfassungseinrichtung kann alternativ oder zusätzlich zur direkten Ermittlung der Lage und/oder des Verlaufs der Blasenkette im Medium bzw. zur Ermittlung von Form und Größe der Blasenkette ausgebildet sein. Zu diesem Zweck kann ein entsprechendes, vorzugsweise optisches Erfassungssystem vorgesehen sein. Dadurch lassen sich in einfacher Weise die Ausbildung und/oder Lage der Blasenkette in dem Medium überwachen und gegebenenfalls Maßnahmen ergreifen, um auf die Lage bzw. den Verlauf der Blasenkette im Medium und/oder die Größe der Blasenkette Einfluß nehmen zu können.

Die Beleuchtungseinrichtung bzw. das Licht kann auf die Blasenkette ausrichtbar sein, um eine Beleuchtung der Blasenkette insbesondere auch bei sich bewegender Blasenkette im Medium sicherzustellen. Beispielsweise kann die Beleuchtungseinrichtung vorzugsweise automatisch auf den Austrittsbereich der Blasenkette ausrichtbar sein, so dass das Licht beispielsweise auch bei wanderndem Aüstrittsbereich der Gasblasen an der Oberflächen zumindest im wesentlichen auf den Austrittsbereich trifft.

Zur Bestimmung des Strömungsprofils und/oder der Strömungsgeschwindigkeit des Mediums kann zusätzlich oder alternativ zur Erfassungseinrichtung eine Meßeinrichtung vorgesehen sein. In diesem Zusammenhang kann eine Steuereinrichtung zur Steuerung der Ausrichtung der Beleuchtungseinrichtung und/oder zur Steuerung der Einrichtung zur Erzeugung einer Bewegung des Mediums in Abhängigkeit von der Lage des Austrittsbereiches der Blasenkette an der Oberfläche des Mediums und/oder von der Lage und/oder der Größe der Blasenkette im Medium und/oder dem Strömungsprofil und/oder der Strömungsgeschwindigkeit des Mediums vorgesehen sein. Damit läßt sich in einfacher Weise auch bei bewegter Blasenkette sicherstellen, dass eine Beleuchtung der Blasenkette mit hoher Beleuchtungsintensität möglich ist. Im übrigen lassen sich Form, Größe und Lage der Blasenkette in einfacher Weise durch Veränderung der Strömungsgeschwindigkeit und/oder des erzeugten Strömungsprofils beeinflussen.

Um eine vorzugsweise rotatorische, kreis- oder spiralförmige Bewegung des Mediums auszubilden, kann ein entsprechendes Mittel, wie Rührwerk vorgesehen sein, wobei, vorzugsweise, die Einleitung von Gas in das Medium zwischen rotierenden Rührelementen des Rührwerks erfolgen kann. Die Rührelemente können von oben in das Medium eintauchen oder von unten, wobei die Gaszuleitung und das Rührwerk auf gleichen oder unterschiedlichen Seiten des Mediums angeordnet sein können. Alternativ oder ergänzend kann sich eine gewünschte Strömung des Mediums auch mit einer Umwälzpumpe erzeugen lassen, mit der das Medium aus einem Mediumbehälter bzw. -tank entnommen bzw. dem Behälter zugeführt oder im Behälter umgewälzt werden kann. Ein Rührwerk muß dann nicht vorgesehen sein.

Eine Beleuchtung der Blasenkette kann zumindest im wesentlichen in vertikaler Richtung vorgesehen sein, beispielsweise ausgehend von einem Bereich oberhalb von dem Medium, wobei das Licht auf den Austrittsbereich der Blasenkette an der Oberfläche des Mediums gerichtet wird. Grundsätzlich ist es aber auch möglich, dass eine Beleuchtung der Blasenkette schräg von oben oder von der Seite des Mediums erfolgt. Ist beispielsweise ein Rührwerk vorgesehen, um das Medium zu bewegen, kann bei rotierenden Rührelementen eine diskontinuierliche Beleuchtung des Mediums zwischen den Rührelementen hindurch vorgesehen sein. Dadurch wird der Aufbau der erfindungsgemäßen Anordnung vereinfacht. Im übrigen läßt sich dadurch die Beleuchtungseinrichtung ausreichend weit von den Rührelementen beabstandet anordnen, so dass ein freier Zugriff sowohl auf das Rührwerk als auch auf die Beleuchtungseinrichtung möglich ist. Generell ist die Beleuchtungseinrichtung vorzugsweise ausreichend weit beabstandet von der Oberfläche des Mediums, so dass eine Verschmutzung der Beleuchtungseinrichtung mit Spritzwasser ausgeschlossen werden kann.

Die Einleitung von Gas in das Medium kann an mehreren unterschiedlichen Stellen erfolgen, wobei auch die Erzeugung mehrerer Blasenketten vorgesehen sein kann. Dadurch läßt sich eine noch bessere Beleuchtung bzw. Beileuchtung des Mediums in verschiedenen oder, allen Raumbereichen des Mediums oder eines Behälters erreichen.

Darüber hinaus kann ein Mediumbehälter bzw. -tank für das Medium vorgesehen sein, wobei der Mediumbehälter Trennwände oder Leitelemente zur Unterteilung des Mediumbehälters in mehrere Kammern (Bereiche) und/oder zur Strömungslenkung aufweisen kann. Durch die Trennwände bzw. Lcitelemente lassen sich einzelne voneinander getrennte Beleuchtungskammern bereitstellen, wobei, vorzugsweise jeder Kammer eine Strahlungsquelle und eine Einrichtung zur Erzeugung einer Bewegung des Mediums in dieser Kammer zugeordnet ist und wobei in jeder Kammer eine Gaseinleitung vorgesehen ist. Im Ergebnis können in dem Mediumbehälter mehrere Blasenketten erzeugt werden, die durch entsprechende Beleuchtung zu einer gleichmäßigen und vollständigen Verteilung des Lichts innerhalb des Mediums führen. Sind Leitelemente in dem Mediumbehälter vorgesehen, können diese ein bestimmtes Strömungsprofil des bewegten Mediums bewirken, um eine gewünschte Blasenkette erzeugen zu können. Die Trennwände und/oder Leitelemente sind vorzugsweise transparent ausgebildet.

Eine vorschlagsgemäße Verwendung der erfindungsgemäßen Anordnung bzw. des erfindungsgemäßen Verfahrens zeichnet sich dadurch aus, dass ein flüssiges Medium mit Algen, wie einer Algensuspension, verwendet wird, wobei Licht in dem Medium dreidimensional verteilt wird und wobei Kohlendioxid in Form von Gasblasen in das Medium eingeleitet wird, um das Wachstum und/oder die Vermehrung der Algen anzuregen und dadurch Biomasse zu bilden. Durch die beleuchtete Blasenkette kann insbesondere eine kontaktlose Lichteinleitung in das Medium mit Beleuchtung des Mediums in einem Mediumbehälter oder -tank erfolgen, was zu einer sehr effektiven Bildung von Biomasse und/oder Bindung von Kohlendioxid führt. Gleichzeitig kommt es zu einer guten Verteilung des Kohlendioxids über die Blasenkette im Medium. Wie bereits erwähnt, kann die Beleuchtung des Mediums von oben, von unten und/oder seitlich erfolgen. Besonders bevorzugt erfolgt die Einleitung des Lichts über die Blasenkette von unten und/oder oben.

Weitere Aspekte, Vorteile, Merkmale und Eigenschaften der vorliegenden Erfmdung ergeben sich aus den Ansprüchen und der nachfolgenden Beschreibung bevorzugter Ausfuhrungsfbnnen anhand der Zeichnung. Es zeigen;
- Fig. 1: eine schematische Darstellung einer vorschlagsgemäßen Anord- nung gemäß einer ersten Ausführungsform,
- Fig. 2: eine schematische Darstellung einer vorschlagsgemäßen Anord- nung gemäß einer zweiten Ausführungsform,
- Fig. 3: eine schematische Darstellung einer vorschlagsgemäßen Anord- nung gemäß einer dritten Ausführungsform,
- Fig. 4: eine schematische Darstellung einer vorschlagsgemäßen Anord- nung gemäß einer vierten Ausführungsform,
- Fig. 5: eine schematische Darstellung einer vorschlagsgemäßen Anord- nung gemäß einer fünften Ausführungsform und
- Fig. 6: eine schematische Darstellung einer vorschlagsgemäßen Anord- nung gemäß einer sechsten Ausführungsform.

In den Figuren werden für gleiche und/oder ähnliche Komponenten, Bauteile und dergleichen die gleichen Bezugszeichen verwendet. Insbesondere ergeben sich entsprechende oder vergleichbare Vorteile, auch wenn eine wiederholte Beschreibung weggelassen ist.

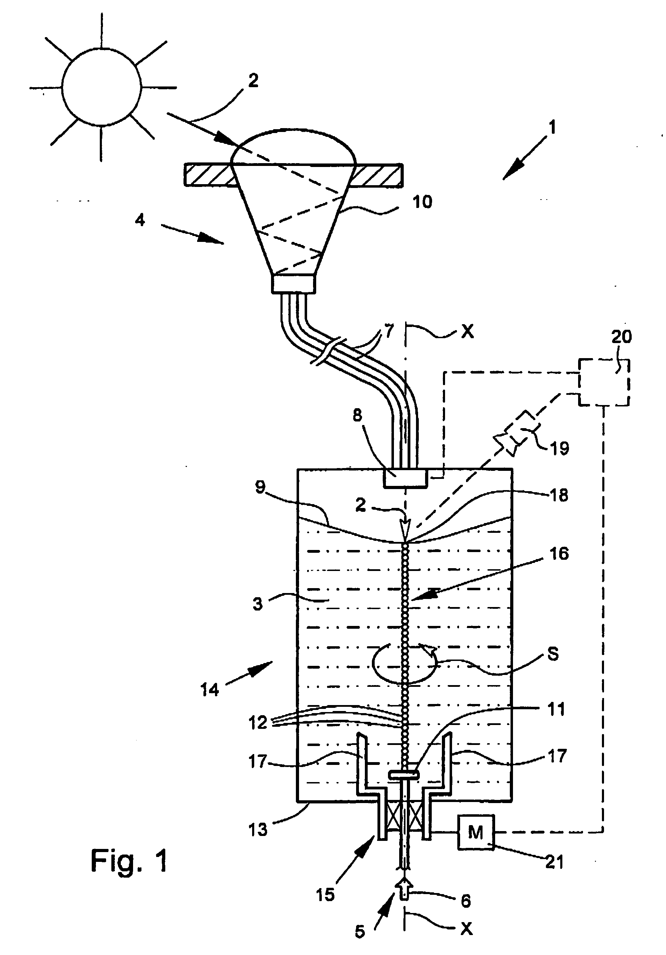

Fig. 1 zeigt in einer schematischen, nicht maßstabsgerechten Darstellung eine vorschlagsgemäße Anordnung 1 gemäß einer ersten Ausführungsform. Die Anordnung 1 dient der insbesondere dreidimensionalen Verteilung von Licht 2 (schematisch in Fig. 1 angedeutet) in einem flüssigen Medium 3. Bei dem Medium 3 handelt es sich insbesondere um eine Suspension o. dgl, Dementsprechend ist der Begriff "flüssig" in einem weiten Sinne so zu verstehen, dass insbesondere Suspensionen, Dispersionen oder sonstige Gemische oder Stoffe mit flüssigen Phasen oder Anteilen umfaßt sind.

Das Medium 3 ist vorzugsweise photoaktiv und/oder biologisch aktiv. Insbesondere kann in dem Medium 3 eine Photosynthese oder eine sonstige Licht 2 benötigende Reaktion ablaufen. Insbesondere enthält das Medium 3 hierzu biologisch aktive Organismen, vorzugsweise Algen, Bakterien o. dgl. Insbesondere ist das Medium 3 stark lichtstreuend.

Vorzugsweise ist das Medium 3 wäßrig bzw. enthält Wasser. Beispielsweise kann das Medium 3 Süßwasser, Brackwasser oder Salzwasser enthalten oder zumindest im wesentlichen daraus bestehen.

Besonders bevorzugt wird eine Algensuspension als Medium 3 eingesetzt. Insbesondere enthält das Medium 3 eine Algenmischkultur, die vorzugsweise zumindest in ähnlicher Form in Flüssen, Teichen o. dgl. vorkommt. Derartige Mischkulturen sind nämlich besonders resistent gegen Umwelteinflüsse, Krankheiten und/oder sonstige Störungen.

Da die Anordnung 1 vorzugsweise mit Algen bzw. mit einer Algensuspension als Medium 3 eingesetzt wird, wird in der nachfolgenden Beschreibung primär auf das durch das eingeleitete Licht 2 induzierte Algenwachstum abgestellt. Jedoch gelten diese Ausführungen entsprechend auch für sonstige Organismen oder photo- bzw. bioaktive Medien 3.

Die in Fig. 1 dargestellte Anordnung 1 weist eine Beleuchtungseinrichtung 4 und eine Einrichtung (Zufuhreinrichtung) 5 zur Einleitung von Gas 6, das insbesondere Kohlendioxid enthält oder daraus besteht, in das Medium 3 auf. Bei dem Gas 6 kann es sich um Rauchgas, Klärgas, Faulgas, Luft o. dgl. handeln. Das Gas 6 kann vorzugsweise einen Anteil von 0,04 % bis 100 % Kohlendioxid enthalten.

Zur Zuleitung von Licht 2 weist die Beleuchtungseinrichtung 4 vorzugsweise lichtleitende Fasern 7 auf, die vorzugsweise in einem Lichtkopf 8 enden. Das Licht 2 tritt zumindest im wesentlichen oder ausschließlich am Ende der Fasern 7 oder am Lichtkopf 8 - insbesondere gebündelt oder gerichtet - aus. Der Lichtkopf 8 bzw. die Beleuchtungseinrichtung 4 ist vorzugsweise oberhalb einer Oberfläche 9 des Mediums 3 angeordnet.

Beim Darstellungsbeispiel wird vorzugsweise Sonnenlicht mit einer Lichtsammeleinrichtung 10, insbesondere einen Kollektor, gesammelt. Es versteht sich, dass eine Beleuchtung auch durch künstliches Licht mit geeigneten Maßnahmen möglich ist

Die Zufuhreinrichtung 5 weist ein geeignetes Einleitungsmittel 11 auf, das in Fig. 1 lediglich schematisch dargestellt ist und aus dem das Gas 6 in Form von Gasblasen 12 austritt. Die Gaseinleitung erfolgt vorzugsweise über oder durch einen Boden 13 eines Mediumbehälters 14, der zur Aufnahme des Mediums 3 dient. Bei dem dargestellten Ausführungsbeispiel ist der Lichtkopf 8 oberhalb des Mediums 3 angeordnet, vorzugsweise gegenüberliegend zur Zufuhreinrichtung 5. Vorzugsweise sind der Lichtkopf 8 und die Zufuhreinrichtung 5 zumindest im wesentlichen koaxial zu einer Rotationsachse X des Mediums 3 angeordnet, worauf nachfolgend noch eingegangen wird.

Die Anordnung 1 weist eine Einrichtung 15 zur Erzeugung einer Blasenkette 16 aus dem in das Medium 3 eingeleiteten Gas 6 bzw. aus in dem Medium 3 aufsteigenden Gasblasen 12 auf, wobei die Blasenkette 16 vorzugsweise mit der Beleuchtungseinrichtung 4 beleuchtbar ist. Die Einrichtung 15 weist vorzugsweise ein Mittel, wie ein Rührwerk, eine entsprechende Umwälzung o. dgl. auf, um das Medium 3 in Bewegung zu setzen, insbesondere um eine rotatorische, kreisförmige oder spiralförmige Strömung S bzw. einen Wirbel oder eine Rotation des Mediums 3 oder in dem Medium 3 zu erzeugen, so dass die Blasenkette 16 gebildet wird. Insbesondere ist der Begriff "Blasenkette" hier als ein Gastunnel bzw. ein für das Licht 2 besonders durchlässiger oder transparenter, primär aus Gas 6 bestehender Bereich innerhalb des Mediums 3 zu verstehen.

Bei der ersten Ausführungsform umfaßt die Einrichtung 15 bzw. das Mittel ein Rührwerk mit insbesondere mehreren Rührelementen 17.

Die Einrichtung 15 ist vorzugsweise unterhalb des Mediumbehälters 14 vorgesehen, wobei die Rührelemente 17 von unten in das Medium 3 eingreifen. Beim Betrieb des Rührwerks wird durch die rotierenden Rührelemente 17 das Medium 3 in eine rotatorische, kreis- oder spiralförmige Bewegung um die Rotationsachse X versetzt, so dass die Blasen 12 zu einer Kette 16 zusammentreten, die sich vorzugsweise zumindest im wesentlichen gerade bzw. linear vorzugsweise vom Boden 13 des Mediumbehälters 14 nach oben erstreckt. In Abhängigkeit von dem Strömungsprofil des Mediums 3 und der Strömungsgeschwindigkeit in dem Mediumbehälter 14 kann sich jedoch auch eine schraubenlinienförmige oder sonstige Blasenkette 16 ausbilden.

Durch die Bewegung des Mediums 3 kommt es auch zu einer Umwälzung bzw. Bewegung der Algenmasse, was für das biologische Wachstum erforderlich oder diesem zuträglich ist.

Der Mediumbehälter 14 weist bei dem in Fig. 1 dargestellten Ausführungsbeispiel vorzugsweise eine Zylinderform mit glatten Innenwänden auf, was der bevorzugten Ausbildung einer gleichmäßigen Rotationsströmung des Mediums 3 um die Rotationsachse X zuträglich ist. Insbesondere ist es von Vorteil, wenn turbulente Strömungen nicht auftreten, die zu einem Abreißen der Blasenkette 16 führen können. Vorzugsweise werden zeitlich nicht oder nur kaum oder nur langsam variierende Strömungsverhältnisse angestrebt.

Alternativ oder zusätzlich kann es jedoch auch vorteilhaft sein, eine turbulente Strömung oder pulsartige Strömung oder Wellen des Mediums 3 - zumindest temporär und/oder bereichsweise - zu erzeugen, beispielsweise um das Wachstum anzuregen und/oder die Durchmischung oder Umwälzung zu fördern.

Vorzugsweise wird in die Blasenkette 16 das aus dem Lichtkopf 8 austretende Licht 2 eingekoppelt, wobei die Blasenkette 16 als Lichtleiter wirkt. Wie bei Wellenleitern lassen sich über die abgestrahlte Intensität als Funktion der Länge die Verluste einer Blasenkette 16 ermitteln, wobei beispielsweise grünes Licht zu einer Dämpfung von ca. 1,5 dB/m geführt hat. Da die Blasen 12 rotieren, erfolgt die Abgabe des Streulichts von der Blasenkette 16 aus radial in alle Richtungen. Die Blasenkette 16 oder ein Blasenstrudel läßt sich dabei grundsätzlich vor allem in optisch dichteren Suspensionen, wie beispielsweise Algensuspensionen, erzeugen und zur Lichtverteilung nutzen.

Um eine kontaktlose Beleuchtung des Mediums 3 - also ohne direkten Kontakt eines Lichtleiters mit dem Medium 3 - zu ermöglichen, erfolgt die Einleitung des Lichts 2 in das Medium 3 vorzugsweise über die Blasenkette 16. Wie sich aus Fig. 1 ergibt, erstreckt sich die Blasenkette 16 im wesentlichen vorzugsweise vom Bodenbereich bis zur Oberfläche 9 des Mediums 3, wobei die Beleuchtung der Blasenkette 16 vorzugsweise im Austrittsbereich 18 der Gasblasen 12 aus dem Medium 3 bzw. an der Oberfläche 9 vorgesehen ist und wobei das Licht 2 vorzugsweise im wesentlichen vertikal und/oder gebündelt auf den Austrittsbereich 18 gerichtet wird. Der Lichtkopf 8 ist vorzugsweise ausreichend weit von der Oberfläche 9 des Mediums 3 beabstandet, um eine Benetzung des Lichtkopfes 8 mit dem Medium 3 ausschließen zu können. Dadurch steht nicht zu befürchten, dass es durch anwachsende Algen auf dem Lichtkopf 8 zu einer Verringerung der Transparenz des Lichtkopfes 8 und der Beleuchtungsstärke kommt.

Vorschlagsgemäß wird die Möglichkeit geschaffen, Licht 2 in optisch dichte Medien 3, wie Suspensionen, einzubringen, wobei eine hohe Lichtintensität in der Blasenkette 16 über eine relativ große Oberfläche ins Medium 3 gelangt und wobei die abgestrahlte Intensität infolge Mehrfachreflexion stark abgeschwächt werden kann, was für viele Algen bzw. deren Wachstum förderlich ist. Durch das Zusammenwirken von Strömung und Rotation der Grenz- bzw. Spiegelflächen der Gasblasen 12 wird eine weitgehend gleichmäßige und vollständige räumliche Verteilung des Lichts 2 im Medium 3 erreicht.

Zur Bestimmung oder Verfolgung des Austrittsbereiches 18 kann eine Erfassungseinrichtung 19 vorgesehen sein, die über eine Steuereinrichtung 20 mit einem Motor 21 des Rührwerks und/oder mit dem Lichtkopf 8 verbunden ist. Der Lichtkopf 8 bzw. das Licht 2 kann sich von der Steuereinrichtung 20 vorzugsweise automatisch auf den erfaßten Austrittsbereich 18 der Blasenkette 16 ausrichten lassen. Insbesondere ist der Lichtkopf 8 entsprechend schwenkbar oder bewegbar, so dass stets eine Einkopplung von Licht 2 in die Blasenkette 16 vorzugsweise über deren Austrittsbereich 18 ermöglicht wird. Dadurch wird auch bei einer aufgrund des bewegten Mediums 3 sich ändernden Lage des Austrittsbereiches 18 stets eine ausreichende Beleuchtung der Blasenkette 16 gewährleistet.

Die Erfassungseinrichtung 19 arbeitet vorzugsweise optisch. Beispielsweise kann die Erfassungseinrichtung 19 eine Kamera aufweisen oder durch diese gebildet sein.

Die Erfassungseinrichtung 19 kann alternativ oder zusätzlich zur Bestimmung oder Verfolgung des Austrittsbereichs 18 auch generell die Lage und/oder Form der Blasenkette 16 bzw. den Verlauf der Gasblasen 12 im Medium 3 erfassen. Jedoch kann hierfür auch zusätzlich eine nicht dargestellte Meβeinrichtung vorgesehen sein.

Alternativ oder zusätzlich kann mittels der Erfassungseinrichtung 19 und/oder der Meßeinrichtung auch eine Bestimmung eines Strömungsprofils und/oder der Strömungsgeschwindigkeit des Mediums 3 erfolgen.

Alle relevanten Meßsignale, Daten und Informationen können von der Steuerung bzw. Steuereinrichtung 20 genutzt werden. Die Steuereinrichtung 20 kann insbesondere die Zuführeinrichtung 5, die Einrichtung 15 und/oder die Lichteinkopplung bzw. den Lichtkopf 8 steuern oder regeln. Beispielsweise kann die Steuerung bzw. Regelung zunächst derart erfolgen, dass die Blasenkette 16 bzw. eine in ihrer Lage stabile Blasenkette 16 erzeugt wird. Weiter kann die Steuerung bzw. Regelung bewirken, dass das Licht 2 immer auf den Austrittsbereich 18 der Blasenkette 16 oder auf einen sonstigen gewünschten Bereich der Blasenkette 16 gerichtet wird oder bleibt, insbesondere bedarfsweise nachgefuhrt wird.

Die Steuereinrichtung 20 kann beispielsweise in Abhängigkeit von der Strömungsgeschwindigkeit und/oder dem Strömungsprofil des Mediums 3 und/oder in Abhängigkeit von dem Auftreten oder der Lage des Austrittsbereiches 18 einen Motor 21 der Einrichtung 15 bzw. des Rührwerks steuern oder regeln, um die Rotationsgeschwindigkeit der Strömung S, des Mediums 3, des Rührwerks bzw. der Rührelemente 17 zu steuern bzw. regeln und ein gewunschtes Strömungsprofil und/oder eine gewünschte Strömungsgeschwindigkeit des Mediums 3 und damit die Ausbildung einer bestimmten Form, Lage und Größe der Blasenkette 16 zu erreichen.

Bei der in Fig. 1 dargestellten Ausführungsform wird Gas 6 zwischen den rotierenden Rührelementen 17 in das Medium 3 eingeleitet Grundsätzlich ist es aber auch möglich, dass eine Gaseinleitung seitlich über oder durch die Wandung des Mediumbehälters 14 erfolgt.

Nachfolgend werden weitere Ausführungsformen der vorliegenden Erfindung anhand der weiteren Figuren näher erläutert, wobei jeweils nur auf wesentliche Unterschiede oder neue Aspekte eingegangen wird. Die bisherigen Ausführungen und Erläuterungen insbesondere bezüglich der ersten Ausführungsform gelten daher vorzugsweise entsprechend oder ergänzend.

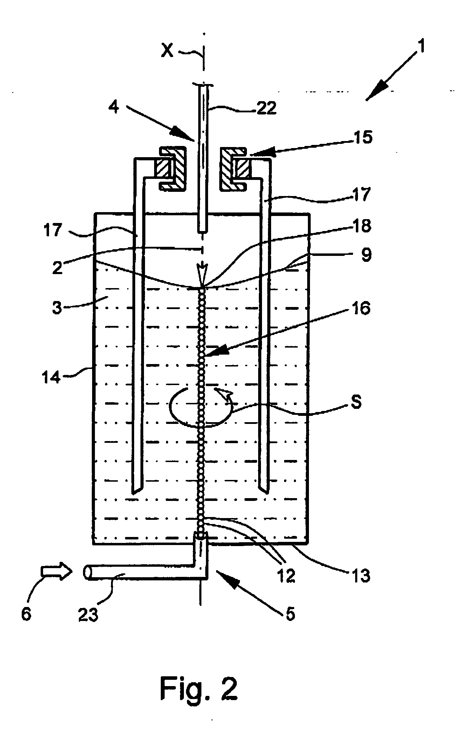

In Fig. 1 ist das Rührwerk unten am oder im Behälter 14 angeordnet. Bei der in Fig. 2 dargestellten zweiten Ausführungsform ist das Rührwerk oben am oder im Mediumbehälter 14 bzw. Medium 3 angeordnet, wobei die Rührelemente 17 von oben in das Medium 3 eingreifen. Gemäß Fig. 2 erstrecken sich die Rührelemente 17 bis in den unteren Bereich des Mediumbehälters 14. Dadurch ist eine gleichmäßige laminare Bewegung des Mediums 3 möglich. Je nach Ausbildung der Rührelemente 17 können diese auch eine kürzere Länge aufweisen. Die Rotationsachse X des Rührwerks bzw. der Einrichtung 15 bzw. der Strömung S verläuft wiederum vorzugsweise vertikal. Die Beleuchtung der Blasenkette 16 erfolgt wiederum im wesentlichen in vertikaler Richtung auf den Austrittsbereich 18 der Gasblasen 12, wobei eine Lichtleitfaser 7 oder ein sonstiger Lichtleiter 22 im Bereich der Rotationsachse X zwischen den Rührelementen 17 hindurchgeführt oder der Lichtkopf 8 dort angeordnet sein kann.

Wie sich aus Fig. 2 weiter ergibt, ist vorzugsweise eine Leitung 23 als Zuführeinrichtung 5 vorgesehen, über die das Gas 6 dem Medium 3 zugeführt wird. Dabei weist die Leitung 23 vorzugsweise lediglich eine Austrittsöffnung auf, aus der das Gas 6 ausströmt und in Form von Gasblasen 12 nach oben aufsteigt. Grundsätzlich können natürlich auch mehrere Gasaustrittsöffnungen, ein Blasenstein, ein Tellerbelüfter o, dgl. vorgesehen sein bzw. zur Gaseinleitung oder als Zuführeinrichtung 5 eingesetzt werden. Beispielsweise können mit einer Art Lochmaske mehrere Blasenketten bzw. Wurzeln bei der Einleitung des Gases 6 in das Medium 3 erzeugt werden, wobei diese mehreren Blasenketten bzw. Wurzeln sich dann insbesondere zu einer einzigen Blasenkette 16 - vorzugsweise in der Drehachse bzw. Rotationsachse X - vereinigen können. Diese Wurzeln können auch zeitlich variieren bzw. sich bewegen. Durch die Wurzeln kann eine größere Verteilung des Gases 6 im Medium 3 im Bodenbereich bzw. in einem unteren Bereich erreicht werden.

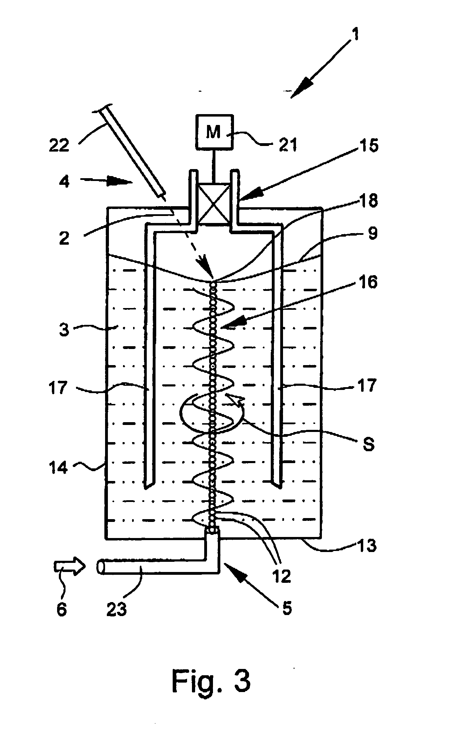

Bei der in Fig. 3 dargestellten dritten Ausführungsform erfolgt die Beleuchtung der Blasenkette 16 schräg von oben bzw. schräg zur Oberfläche 9 des Mediums 3, vorzugsweise wobei bei rotierenden Rührelementen 17 eine diskontinuierliche Beleuchtung des Mediums 3 zwischen den Rührelementen 17 hindurch vorgesehen ist.

Bei der dritten Ausführungsform gemäß Fig. 3 ist schematisch angedeutet, dass die Blasenkette 16 anstelle eines zumindest im wesentlichen geradlinigen Verlaufs auch einen schraubenlinienförmigen oder helixartigen Verlauf aufweisen kann.

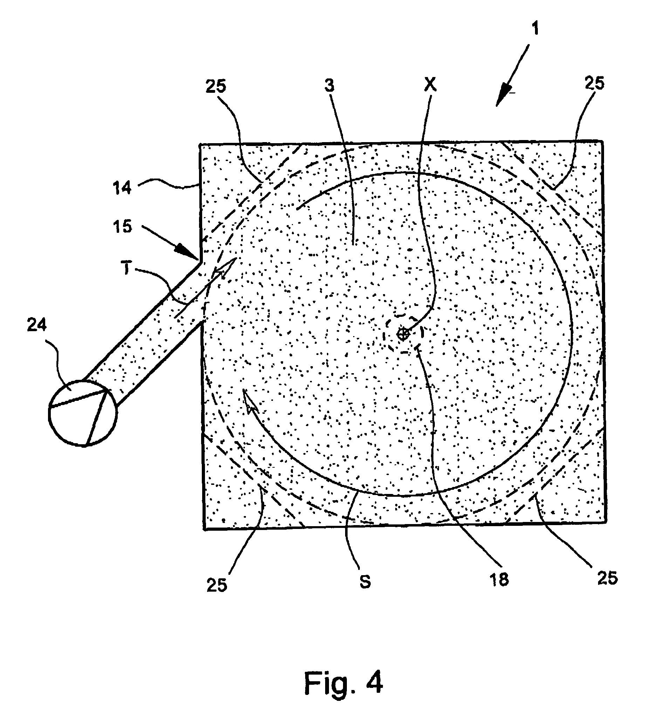

Figuren 1 bis 3 zeigen bevorzugte Ausführungsformen der vorschlagsgemäßen Anordnung 1 jeweils in einem schematischen Vertikalschnitt. Figuren 4 bis 6, die nachfolgend besprochen werden, zeigen jeweils bevorzugte Ausführungsformen der vorschlagsgemäßen Anordnung 1 in einem schematischen Horizontalschnitt bzw. in schematischer Draufsicht.

Bei der vierten Ausführungsform gemäß Fig. 4 weist die Einrichtung 15 bzw. das Mittel zur Erzeugung einer Bewegung bzw. der Strömung S des Mediums 3 alternativ oder zusätzlich zu dem Rührwerk eine Pump- und/oder Umwälzeinrichtung, hier eine Umwälzpumpe 24, auf. Bei der vierten Ausführungsform wird das Medium 3 vorzugsweise schräg bzw. in tangentialer Richtung, wie durch Pfeil T angedeutet, in den Behälter 14 bzw. einen Bereich des Behälters 14, wo die Strömung S erzeugt werden soll, eingeleitet, um die gewünschte, insbesondere rotatorische Bewegung oder Strömung S zu erzeugen bzw. die Blasenkette 16 zu bilden.

Der Mediumbehälter 14 ist gemäß Fig. 4 vorzugsweise viereckig ausgebildet. Er kann Leitelemente 25 aufweisen, die die Ausbildung einer Dreh-, Wirbel- bzw. Rotationsströmung S im Mediumbehälter 14 unterstützen. Die Leitelemente 25 können beispielsweise die Strömung S an Ecken des Behälters 14 vorbeileiten. Hierzu können die Leitelemente 25 beispielsweise gerade oder gebogen ausgebildet sein, wie gestrichelt in Fig. 4 angedeutet. Alternativ und zusätzlich kann der Behälter 14 statt der viereckigen oder quadratischen Querschnittsform auch eine sonstige Form, beispielsweise eine polygonale Form, insbesondere eine regelmäßige Mehreckform, insbesondere eine Sechseck- oder Achteckform, oder auch eine runde Form, insbesondere Kreisform, aufweisen, wie ebenfalls angedeutet.

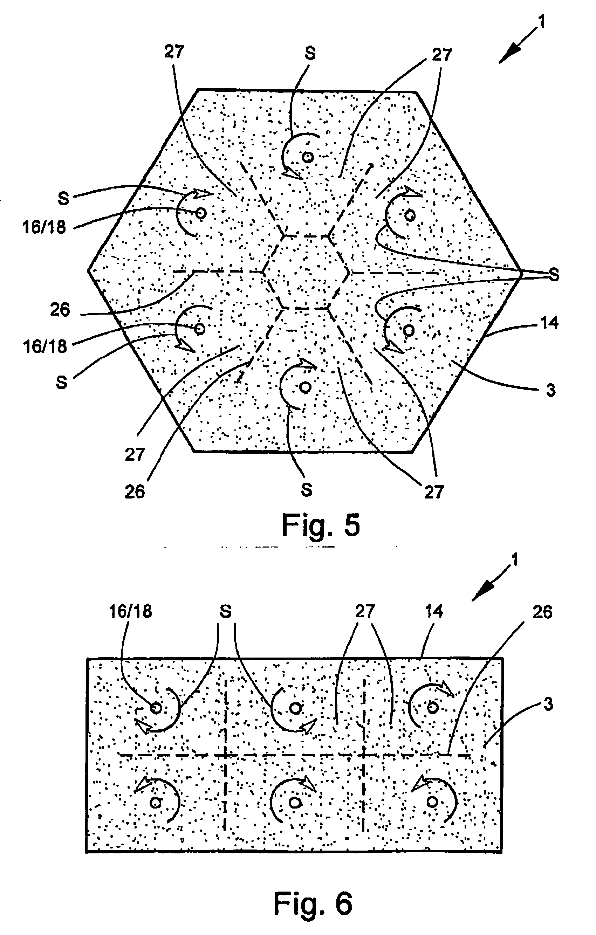

Bei der in Fig. 5 dargestellten Ausführungsform weist der Mediumbehälter 14 vorzugsweise eine sechseckige oder sonstige mehreckige Querschnittsform auf. Grundsätzlich kann der Mediumbehälter 14 aber auch ein anderes polygonales oder sonstiges Querschnittsprofil aufweisen oder zylinderförmig ausgebildet sein.

Vorzugsweise werden in dem Mediumbehälter 14 mehrere Blasenketten 16 erzeugt, insbesondere in unterschiedlichen Bereichen 27, die bedarfsweise durch Leitelemente 25 und/oder Trennwände 26 voneinander getrennt sein können. Jedem Bereich 27 kann dabei eine eigene Strahlungsquelle bzw. Beleuchtungseinrichtung und/oder eine Einrichtung 15 zur Erzeugung der Strömung S des Mediums 3 zugeordnet sein. Darüber hinaus kann in jeder Kammer 27 eine Gaseinleitung über oder durch den Boden vorgesehen sein. Dies ist in Fig. 5 lediglich schematisch angedeutet. Grundsätzlich können mehrere Blasenketten 16 in einem Mediumbehälter 14 erzeugt werden, wobei in dem Mediumbehälter 14 ein nicht-unterbrochener Strömungsraum für das Medium 3 gebildet sein kann. Die verschiedenen Bereiche 27 bzw. Blasketten 16 sind dann vorzugsweise ausreichend weit voneinander entfernt, insbesondere um zumindest im wesentlichen eine gegenseitige Störung zu vermeiden.

Durch die Mehrzahl von beleuchteten Blasenketten 16 in dem Mediumbehälter 14 wird eine besonders gleichmäßige, verteilte und/oder vollständige Ausleuchtung des Mediums 3 ermöglicht.

Fig. 6 zeigt in einer schematischen Darstellung eine sechste Ausführungsform der vorliegenden Anordnung 1. Hier sind in einem beispielsweise rechteckigen oder sonstigen, vorzugsweise mehreckigen Behälter 3 mehrere Bereiche 27 gebildet, in denen jeweils eine Blasenkette 16, insbesondere auf Grund einer entsprechenden, vorzugsweise rotatorischen oder wirbelartigen Strömung S erzeugt werden. Einzelne oder alle Blasenketten 16 können beleuchtet werden, um Licht in das Medium 3 im Behälter 14 einzuleiten und insbesondere darin dreidimensional zu verteilen. Die einzelnen Bereiche 27 können durch beispielsweise gitterartig angeordnete Leitelemente 25 oder Trennwände 26 o. dgl. zumindest partiell oder soweit erforderlich voneinander abgetrennt sein.

Einzelne Merkmale und Aspekte der voranstehend beschriebenen Ausführungsformen und der eingangs beschriebenen Erfindung können unabhängig voneinander, aber auch in beliebiger Kombination realisiert werden.

### Bezugszeichen:

- 1: Anordnung
- 2: Licht
- 3: Medium
- 4: Beleuchtungseinrichtung
- 5: Zufthreinrichtung
- 6: Gas
- 7: Faser
- 8: Lichtkopf
- 9: Oberfläche
- 10: Lichtsammeleinrichtung
- 11: Einleitungsmittel
- 12: Gasblase
- 13: Boden
- 14: Mediumbehälter
- 15: Einrichtung
- 16: Blasenkette
- 17: Rührelement
- 18: Austrittsbereich
- 19: Erfassungseinrichtung
- 20: Steuereinrichtung
- 21: Motor
- 22: Lichtleiter
- 23: Leitung
- 24: Umwälzpumpe
- 25: Leitelement
- 26: Trennwand
- 27: Kammer

## Patentansprüche

1. Anordnung (1) zur dreidimensionalen Verteilung von Licht (2) in einem flüssigen Medium (3), das vorzugsweise Organismen, insbesondere Algen, enthält, mit einer Beleuchtungseinrichtung (4) und mit einer Zuführeinrichtung (5) zur Einleitung von Gas (6) in das Medium (3), wobei eine Einrichtung (15) zur Erzeugung einer Blasenkette (16) von in das Medium (3) eingeleitetem Gas (6) vorgesehen ist, wobei die Einrichtung (15) zur Erzeugung einer rotatorischen oder kreis- oder spiralförmigen Bewegung der Strömung (S) des Mediums (3) ausgebildet ist und sich Gasblasen (12) im Bereich der Rotationsachse sammeln und die Blasenkette (16) bilden, wobei die Blasenkette (16) mit der Beleuchtungseinrichtung (4) zur Einleitung von Licht (2) in die Blasenkette (16) beleuchtbar ist.

2. Anordnung nach Anspruch 1, **dadurch gekennzeichnet, dass** sich die Blasenkette (16) zumindest im Wesentlichen durchgängig bis zur Oberfläche (9) des Mediums (3) erstreckt.

3. Anordnung nach einem der vorhergehenden Ansprüche, **dadurch gekennzeichnet, dass** eine mit dem Medium (3) kontaktlose Beleuchtung der Blasenkette (16) vorgesehen ist von einem Bereich außerhalb des Mediums (3).

4. Anordnung nach einem der vorhergehenden Ansprüche, **dadurch gekennzeichnet, dass** eine Erfassungseinrichtung (19) zur Bestimmung, Erfassung oder Verfolgung des Austrittsbereiches (18) der Blasenkette (16) an der Oberfläche (9) des Mediums (3) und/oder der Lage und/oder Größe der Blasenkette (16) vorgesehen ist.

5. Anordnung nach einem der vorhergehenden Ansprüche, **dadurch gekennzeichnet, dass** die Beleuchtungseinrichtung (4) bzw. das Licht (2) auf die Blasenkette (16) ausrichtbar ist.

6. Anordnung nach einem der vorhergehenden Ansprüche, **dadurch gekennzeichnet, dass** eine Beleuchtung der Blasenkette (16) zumindest im wesentlichen in vertikaler Richtung, von oben und/oder von unten vorgesehen ist.

7. Anordnung nach einem der vorhergehenden Ansprüche, **dadurch gekennzeichnet, dass** eine Steuereinrichtung (20) zur Steuerung oder Regelung der Ausrichtung der Beleuchtungseinrichtung (4) und/oder zur Steuerung oder Regelung der Einrichtung (15) zur Erzeugung einer Blasenkette (16) bzw. Bewegung des Mediums (3) und/oder Steuerung oder Regelung der Gaszufuhr bzw. der Zufuhreinrichtung (5) - insbesondere in Abhängigkeit von dem Austrittsbereich (18) der Blasenkette (16) an der Oberfläche (9) des Mediums (3) und/oder der Lage und/oder der Größe der Blasenkette (16) und/oder dem Strömungsprofil und/oder der Strömungsgeschwindigkeit des Mediums (3) - vorgesehen ist.

8. Anordnung nach einem der vorhergehenden Ansprüche, **dadurch gekennzeichnet, dass** die Einrichtung (15) zur Erzeugung einer Blasenkette (16) bzw. Bewegung des Mediums (3) ein Rührwerk aufweist, wobei, vorzugsweise, die Einleitung von Gas (6) in das Medium (3) zwischen rotierenden Rührelementen (17) des Rührwerks vorgesehen ist.

9. Anordnung nach einem der voranstehenden Ansprüche, **dadurch gekennzeichnet, dass** die Einrichtung (15) zur Erzeugung einer Blasenkette (16) bzw. Bewegung des Mediums (3) eine Umwälz- oder Pumpeinrichtung, insbesondere eine Umwälzpumpe (24), aufweist und/oder zur zumindest im wesentlichen tangentialen Einleitung eines Mediumstroms in einen das Medium (3) enthaltenden Behälter (14) oder Bereich (27) ausgebildet ist

10. Anordnung nach einem der vorhergehenden Ansprüche, **dadurch gekennzeichnet, dass** eine Beleuchtung der Blasenkette (16) schräg zur Oberfläche (9) des Mediums (3) und, vorzugsweise, bei rotierenden Rührelementen (17) eine diskontinuierliche Beleuchtung des Mediums (3) zwischen den Rührelementen (17) hindurch vorgesehen sind.

11. Anordnung nach einem der vorhergehenden Ansprüche, **dadurch gekennzeichnet, dass** die Einleitung von Gas (6) in das Medium (3) an mehreren unterschiedlichen Stellen erfolgt, insbesondere so dass mehrere Blasenketten (16) erzeugbar sind.

12. Anordnung nach einem der vorhergehenden Ansprüche, **dadurch gekennzeichnet, dass** ein Mediumbehälter (14) für das Medium (3) vorgesehen ist und dass der Mediumbehälter (14) Trennwände (26) oder Leitelemente (25) zur Unterteilung des Mediumbehälters (14) in mehrere Kammern (27) und/oder zur Strömungslenkung aufweist.

13. Anordnung nach einem der vorhergehenden Ansprüche, **dadurch gekennzeichnet, dass** jeder Kammer (27) eine Strahlungsquelle und eine Einrichtung (15) zur Erzeugung einer Bewegung des Mediums (3) in der Kammer (27) zugeordnet ist und dass in jeder Kammer (27) eine Gaseinleitung vorgesehen ist.

14. Verfahren zur dreidimensionalen Verteilung von Licht (2) in einem flüssigen Medium (3), das vorzugsweise Organismen, insbesondere Algen, enthält,
**dadurch gekennzeichnet,**
**dass** eine Blasenkette (16) von in das Medium (3) eingeleitetem Gas (6) mittels einer rotatorischen, kreisförmigen oder spiralförmigen Bewegung des Mediums (3) erzeugt wird und sich Gasblasen (12) im Bereich der Rotationsachse sammeln und die Blasenkette (16) bilden, wobei Licht (2) über die Blasenkette (16) in das Medium (3) eingeleitet wird.

15. Verfahren nach Anspruch 14, **dadurch gekennzeichnet, dass** eine mit dem Medium (3) kontaktlose Beleuchtung durch die Blasenkette (16) erfolgt.

## Claims

1. An assembly (1) for distributing light (2) in three dimensions in a liquid medium (3) preferably containing organisms, in particular algae, comprising a lighting device (4) and a supply device (5) for introducing gas (6) into the medium (3), wherein a device (15) for generating a bubble chain (16) of gas (6) introduced into the medium (3) is provided, wherein the device (15) is made for generating a rotating or circular or spiral motion of the flow (S) of the medium (3), and gas bubbles (12) collect in the area of the rotational axis and form the bubble chain (16), wherein the bubble chain (16) can be illuminated by the lighting device (4) for introducing light (2) into the bubble chain (16).

2. The assembly according to claim 1, **characterized in that** the bubble chain (16) extends at least substantially continuously up to the surface (9) of the medium (3).

3. The assembly according to any of the preceding claims, **characterized in that** lighting of the bubble chain (16) without contact with the medium (3) is provided from an area outside the medium (3).

4. The assembly according to any of the preceding claims, **characterized in that** a detection device (19) is provided for determining, detecting, or tracking the exit area (18) of the bubble chain (16) at the surface (9) of the medium (3) and/or the situation and/or the dimension of the bubble chain (16).

5. The assembly according to any of the preceding claims, **characterized in that** the lighting device (4) or the light (2) can be aligned on the bubble chain (16).

6. The assembly according to any of the preceding claims, **characterized in that** lighting of the bubble chain (16) is provided at least substantially in the vertical direction, from above and/or from below.

7. The assembly according to any of the preceding claims, **characterized in that** a control device (20) for controlling or adjusting the alignment of the lighting device (4) and/or controlling or adjusting the device (15) for generating a bubble chain (16) or moving the medium (3) and/or controlling or adjusting the gas supply or supply device (5) - in particular depending on the exit area (18) of the bubble chain (16) at the surface (9) of the medium (3) and/or the situation and/or the dimension of the bubble chain (16) and/or the flow profile and/or the flow velocity of the medium (3).

8. The assembly according to any of the preceding claims, **characterized in that** the device (15) for generating a bubble chain (16) or moving the medium (3) has an agitating mechanism, wherein preferably the introduction of gas (6) into the medium (3) is provided between rotating agitators (17) of the agitating mechanism.

9. The assembly according to any of the preceding claims, **characterized in that** the device (15) for generating a bubble chain (16) or moving the medium (3) has a circulating or pumping device, in particular a circulation pump (24) and/or is made for at least substantially tangential introduction of a medium flow into a container (14) or area (27) containing the medium (3).

10. The assembly according to any of the preceding claims, **characterized in that** lighting of the bubble chain (16) is provided obliquely to the surface (9) of the medium (3), and preferably discontinuous lighting of the medium (3) between the agitators (17) is provided when the agitators (17) are rotating.

11. The assembly according to any of the preceding claims, **characterized in that** the introduction of gas (6) into the medium (3) is performed at several different locations, in particular so that several bubble chains (16) can be generated.

12. The assembly according to any of the preceding claims, **characterized in that** a medium container (14) is provided for the medium (3), and **in that** the medium container (14) has separating walls (26) or guide elements (25) for dividing the medium container (14) into several chambers (27) and/or for flow steering.

13. The assembly according to any of the preceding claims, **characterized in that** each chamber (27) is assigned a radiation source and a device (15) for generating a motion of the medium (3) inside the chamber (27), and **in that** gas introduction is provided in each chamber (27).

14. A method for distributing light (2) in three dimensions in a liquid medium (3) preferably containing organisms, in particular algae,
**characterized in**
**that** a bubble chain (16) is generated from gas (6) introduced into the medium (3) by means of a rotating, circular or spiral motion of the medium (3), and gas bubbles (12) collect in the area of the rotational axis and form the bubble chain (16), with light (2) being introduced into the medium (3) via the bubble chain (16).

15. The method according to claim 14, **characterized in that** lighting of the bubble chain (16) is done without contact with the medium (3).

## Revendications

1. Ensemble (1) de distribution de lumière (2) en trois dimensions dans un milieu liquide (3) qui contient de préférence des organismes, en particulier des algues, comprenant un dispositif d'éclairage (4) et un dispositif d'alimentation (5) pour introduire un gaz (6) dans le milieu (3), dans lequel un dispositif (15) pour générer une série de bulles (16) d'un gaz (6) introduit dans le milieu (3) est prévu, dans lequel le dispositif (15) est réalisé pour générer un mouvement rotatif ou circulaire ou en spirale de l'écoulement (S) du milieu (3), et des bulles de gaz (12) s'accumulent dans la zone de l'axe de rotation et forment la série de bulles (16), dans lequel la série de bulles (16) peut être éclairée par le dispositif d'éclairage (4) pour introduire de la lumière (2) dans la série de bulles (16).

2. Ensemble selon la revendication 1, **caractérisé en ce que** la série de bulles (16) s'étend au moins substantiellement en continu jusqu'à la surface (9) du milieu (3).

3. Ensemble selon l'une quelconque des revendications précédentes, **caractérisé en ce qu'**un éclairage de la série de bulles (16) sans contact avec le milieu (3) est prévu à partir d'une zone en dehors du milieu (3).

4. Ensemble selon l'une quelconque des revendications précédentes, **caractérisé en ce qu'**un dispositif de détection (19) est prévu pour déterminer, détecter ou suivre la zone de sortie (18) de la série de bulles (16) à la surface (9) du milieu (3) et/ou la situation et/ou dimension de la série de bulles (16).

5. Ensemble selon l'une quelconque des revendications précédentes, **caractérisé en ce que** le dispositif d'éclairage (4) ou la lumière (2) peut être aligné(e) sur la série de bulles (16).

6. Ensemble selon l'une quelconque des revendications précédentes, **caractérisé en ce qu'**un éclairage de la série de bulles (16) est prévu au moins substantiellement dans la direction verticale, par le haut et/ou par le bas.

7. Ensemble selon l'une quelconque des revendications précédentes, **caractérisé en ce qu'**un dispositif de commande (20) est prévu pour commander ou réguler l'alignement du dispositif d'éclairage (4) et/ou commander ou réguler le dispositif (15) pour générer une série de bulles (16) ou déplacer le milieu (3) et/ou commander ou réguler l'alimentation en gaz ou le dispositif d'alimentation (5) - en particulier en fonction de la zone de sortie (18) de la série de bulles (16) à la surface (9) du milieu (3) et/ou la situation et/ou la dimension de la série de bulles (16) et/ou le profil d'écoulement et/ou la vitesse d'écoulement du milieu (3).

8. Ensemble selon l'une quelconque des revendications précédentes, **caractérisé en ce que** le dispositif (15) pour générer une série de bulles (16) ou déplacer le milieu (3) comporte un agitateur, dans lequel de préférence l'introduction de gaz (6) dans le milieu (3) est prévue entre des pales agitatrices rotatives (17) de l'agitateur.

9. Ensemble selon l'une quelconque des revendications précédentes, **caractérisé en ce que** le dispositif (15) pour générer une série de bulles (16) ou déplacer le milieu (3) présente un dispositif de recirculation ou de pompage, en particulier une pompe de recirculation (24) et/ou est réalisé pour une introduction au moins substantiellement tangentielle d'un écoulement de milieu dans un réservoir (14) ou une zone (27) contenant le milieu (3).

10. Ensemble selon l'une quelconque des revendications précédentes, **caractérisé en ce qu'**un éclairage de la série de bulles (16) est prévu de manière oblique par rapport à la surface (9) du milieu (3), et de préférence, lorsque les pales agitatrices (17) tournent, un éclairage discontinu du milieu (3) est prévu entre les pales agitatrices (17).

11. Ensemble selon l'une quelconque des revendications précédentes, **caractérisé en ce que** l'introduction de gaz (6) dans le milieu (3) est effectuée à plusieurs endroits différents, en particulier de telle sorte que plusieurs séries de bulles (16) peuvent être générées.

12. Ensemble selon l'une quelconque des revendications précédentes, **caractérisé en ce qu'**un réservoir de milieu (14) est prévu pour le milieu (3), et **en ce que** le réservoir de milieu (14) présente des cloisons (26) ou éléments de guidage (25) pour diviser le réservoir de milieu (14) en plusieurs chambres (27) et/ou pour diriger l'écoulement.

13. Ensemble selon l'une quelconque des revendications précédentes, **caractérisé en ce que** chaque chambre (27) se voit attribuer une source de rayonnement et un dispositif (15) pour générer un mouvement du milieu (3) à l'intérieur de la chambre (27), et **en ce qu'**une introduction de gaz est prévue dans chaque chambre (27).

14. Procédé de distribution de lumière (2) en trois dimensions dans un milieu liquide (3) contenant de préférence des organismes, en particulier des algues,
**caractérisé en ce**
**qu'**une série de bulles (16) est générée à partir d'un gaz (6) introduit dans le milieu (3) au moyen d'un mouvement rotatif, circulaire ou en spirale du milieu (3), et des bulles de gaz (12) s'accumulent dans la zone de l'axe de rotation et forment la série de bulles (16), la lumière (2) étant introduite dans le milieu (3) par l'intermédiaire de la série de bulles (16).

15. Procédé selon la revendication 14, **caractérisé en ce qu'**un éclairage de la série de bulles (16) est effectué sans contact avec le milieu (3).
